(19) Europäisches Patentamt / European Patent Office / Office européen des brevets

(11) **EP 1 786 398 B1**

(12) **EUROPEAN PATENT SPECIFICATION**

(45) Date of publication and mention of the grant of the patent:
**12.08.2015 Bulletin 2015/33**

(51) Int Cl.:
*A61K 9/06* *(2006.01)*  *A61K 31/568* *(2006.01)*
*A61K 47/10* *(2006.01)*  *A61K 47/32* *(2006.01)*
*A61K 47/38* *(2006.01)*  *A61K 47/14* *(2006.01)*
*A61K 47/18* *(2006.01)*

(21) Application number: **05794868.9**

(22) Date of filing: **07.09.2005**

(86) International application number:
**PCT/EP2005/010293**

(87) International publication number:
**WO 2006/027278 (16.03.2006 Gazette 2006/11)**

(54) **TESTOSTERONE GELS COMPRISING PROPYLENE GLYCOL AND ISOPROPYL MYRISTATE AS PENETRATION ENHANCERS**

TESTOSTERONGELE MIT PROPYLENGLYKOL UND ISOPROPYLMYRISTAT ALS PENETRATIONSVERBESSERER

GELS A BASE DE TESTOSTERONE COMPORTANT DU PROPYLENE GLYCOL ET DU MYRISTATE D'ISOPROPYLE EN TANT QUE PROMOTEURS DE PENETRATION

(84) Designated Contracting States:
**AT BE BG CH CY CZ DE DK EE ES FI FR GB GR HU IE IS IT LI LT LU LV MC NL PL PT RO SE SI SK TR**

(30) Priority: **09.09.2004 EP 04292170**
**23.12.2004 US 638360 P**

(43) Date of publication of application:
**23.05.2007 Bulletin 2007/21**

(73) Proprietor: **Besins Healthcare Luxembourg SARL 2668 Luxembourg (LU)**

(72) Inventor: **SALIN-DROUIN, Dominique**
**F-91370 Verrières-le-Buisson (FR)**

(74) Representative: **Brown, David Leslie**
**Haseltine Lake LLP**
**Redcliff Quay**
**120 Redcliff Street**
**Bristol BS1 6HU (GB)**

(56) References cited:
WO-A-99/20257       WO-A-02/051421
WO-A-03/047548       WO-A-03/088974

## Description

The present invention relates to testosterone-containing pharmaceutical compositions and gels, and to methods using the same.

## Testosterone in Men

Testosterone, the major circulating androgen in men, is synthesized from cholesterol. The approximately 500 million Leydig cells in the testes secrete more than 95% of the 6-7 mg of testosterone produced per day. Two hormones produced by the pituitary gland, luteinizing hormone ("LH") and follicle stimulating hormone ("FSH"), are required for the development and maintenance of testicular function and negatively regulate testosterone production. Circulating testosterone is metabolized to various 17-keto steroids through two different pathways. Testosterone can be metabolized to dihydrotestosterone ("DHT") by the enzyme 5-alpha-reductase or to estradiol ("E2") by an aromatase enzyme complex. Testosterone circulates in the blood 98% bound to protein. In men, approximately 40% of the binding is to the high-affinity sex hormone binding globulin ("SHBG"). The remaining 60% is bound weakly to albumin. Thus, a number of measurements for testosterone are available from clinical laboratories.

The following table from the UCLA-Harbor Medical Center summarizes the hormone concentrations in normal adult men:

Hormone Levels in Normal Men : Hormone Normal Range

| | |
|---|---|
| Testosterone (total) | 298 to 1043ng/dL |
| Free Testosterone | 3.5 to 17.9ng/dL |
| DHT | 31 to 193ng/dL |
| DHT/T Ratio | 0.052 to 0.33 |
| DHT + T | 372 to 1349ng/dL |
| SHBG | 10.8 to 46.6nmol/L |
| FSH | 1.0 to 6.9mlU/mL |
| LH | 1.0 to 8.1 mlU/mL |
| E2 | 17.1 to 46.1 pg/mL |

There is considerable variation in the half-life of testosterone reported in the literature, ranging from 10 to 100 minutes. Researchers do agree, however, that circulating testosterone has a diurnal variation in normal young men. Maximum levels occur at approximately 6:00 to 8:00 a.m. with levels declining throughout the day. Characteristic profiles have a maximum testosterone level of 720 ng/dL and a minimum level of 430 ng/dL. The physiological significance of this diurnal cycle, if any, however, is not clear.

## Testosterone in Women

The excretion of androgenic steroids in the urine of adult women was demonstrated more than 50 years ago. Since that time, physiologists and clinicians have explored the sources and biological functions of testosterone and other endogenous androgenic hormones in the human female. It is now known that androgens are secreted by both the ovaries and adrenal glands in women. Each source contributes about 50% (directly and through precursors) to the approximately 300 μg of testosterone produced daily in healthy "cycling" women. While the adverse effects of excess androgen production, as occurs in the polycystic ovary syndrome and certain androgen producing tumors, have been well described, the normal physiological effects of androgens in women have been much less appreciated. As inferred from animal studies, male physiology, and the symptoms of women with deficient androgen production, the major physiological effects of androgens in normal women include, but are not limited to anabolic effects on muscle, skin, hair and bone; stimulatory effects on erythropoiesis; modulatory effects on immune function; and psychological effects on mood, well-being and sexual function.

In addition, endogenous androgens are important for the development of pubic hair and are thought to modulate the action of estrogens and progestins on a variety of reproductive target tissues. It is also believed that androgens play an important role in modulating the secretory function of the lacrimal gland.

Fifty percent of circulating testosterone is derived from direct ovarian secretion in the thecal cells under the control of luteinizing hormone. The other half is derived from peripheral conversion of adrenal androgen precursors dehydroepiandrosterone, androstenedione, and dehydroepiandrosterone sulphate. Testosterone can also be converted to dihydrotestosterone or estradiol. Thus, testosterone serves as both a hormone and as a pro-hormone.

Testosterone circulates in the blood 98% bound to protein. In women, approximately 66% of the binding is to the high-affinity sex hormone binding globulin. The remaining 34% is bound weakly to albumin. Thus, a number of measurements for testosterone are available from clinical laboratories. The order of affinity for the steroids most strongly bound by sex hormone binding globulin is dihydrotestosterone > testosterone > androstenedione > estradiol > estrone. Sex hormone binding globulin weakly binds dihydrotestosterone, but not dihydrotestosterone sulfate. The table below shows the approximate hormonal levels in normal pre-menopausal women.

Hormone Levels in Normal Pre-Menopausal Women

| Hormone | Mean +/- sd | Median | Range |
|---|---|---|---|
| Testosterone (nmoL/L) | 1.20 +/- 0.69 | 0.98 | 0.4-2.7 |
| Free testosterone (pmol/L) | 12.80 +/- 5.59 | 12.53 | 4.1-24.2 |
| % Free testosterone of total testosterone | 1.4 +/- 1.1 | 1.1 | 0.4-6.3 |
| Luteinizing hormone (IU/L) | 7.2 +/- 3.3 | 6.7 | 3.0-18.7 |
| Follicle stimulating hormone (IU/L) | 4.7 +/- 3.6 | 4.2 | 1.5-21.4 |
| Sex hormone binding globulin (nmol/L) | 66.1 +/- 22.7 | 71.0 | 17.8-114.0 |

In comparison to other hormone deficiency states, testosterone deficiency in women has been largely ignored as a clinical entity. Nevertheless, there exist well-defined subject populations where androgen production is clearly deficient and where associated symptomatology has been described, including, for example, young oophorectomized/hysterectomized women, post-menopausal women on estrogen replacement therapy, women on oral contraceptives, women with adrenal dysfunction, women with corticosteroid-induced adrenal suppression, and human immunodeficiency virus-positive women.

Because increasing testosterone concentrations has been shown to alter sexual performance and libido, researchers have investigated methods of delivering testosterone to men, and also to women. These methods include intramuscular injections, oral replacement, pellet implants, and transdermal patches.

These testosterone delivery methods, however, suffer from one or more drawbacks. For example, sub-dermal pellet implants and ester injections are painful and require surgical procedure and/or doctor visits. Moreover, in hypogonadal men implant therapy includes a risk of extrusion (8.5%), bleeding (2.3%), or infection (0.6%). Many of these methods, such as oral/sublingual/buccal preparations, suffer from undesirable pharmacokinetic profile-creating supraphysiologic testosterone concentrations followed a return to baseline. Transdermal patches provide less than optimal pharmacokinetic characteristics, are embarrassing for many subjects, and are associated with significant skin irritation. In addition, patches do not provide dosing flexibility, might be visually unappealing and may have a tendency to fall off, especially during rigorous physical exercise. Oral administration produces inappropriate testosterone levels and unpredictable absorption patterns between subjects. Moreover, because the liver metabolizes the preparation, there is a risk of hepatotoxicity not to mention first pass metabolism.

Recently, a one-percent testosterone gel has been approved for use in men, and provides dosing flexibility with minimal skin irritation. This gel is available in the United States under the trademark AndroGel® from Unimed Pharmaceuticals, Inc., Deerfield, Ill., a SolvayPharmaceuticals, Inc.Co. , Marietta, GA 30062.

Composition of AndroGel ®:

| Composition of AndroGel ®: SUBSTANCE | AMOUNT (w/w) PER 100 g OF GEL |
|---|---|
| Testosterone | 1.0 g |
| Carbopol 980 | 0.90 g |
| Isopropyl myristate | 0.50 g |
| 0.1 N NaOH | 4.72 g |

(continued)

| Composition of AndroGel ®: SUBSTANCE | AMOUNT (w/w) PER 100 g OF GEL |
|---|---|
| Ethanol (95% v/v) | 72.5 g* |
| Purified water (qsf) | 100 g |
| *corresponding to 67 g of absolute ethanol. | |

Patent application WO 2003/088974 discloses a pharmaceutical composition, preferably in the form of a gel, comprising an androgen; a cyclic enhancer, a thickening agent.

Document WO 2002/051421 discloses a gel composition comprising at least one androgenic steroid and at least one C3 to C4 diol as resorption increasing agent.

Patent application WO 1999/020257 discloses a hormonal drug containing alcoholic or aqueous alcoholic composition which comprises a hormonally active drug; a skin penetration enhancing effective amount of a C7 to C14-hydrocarbyl substituted 1,3-dioxolane, 1,3-dioxane or acetal, 1,2-propylene glycol; a volatile alcohol selected from ethanol, isopropanol and mixture thereof; water; and, optionally, a gelling agent.

International application WO 2003/047548 discloses compositions comprising dihydroxytestosterone (DHT) and a penetration enhancer such as isopropyl myristate. The composition can further comprise ethanol and/or a gelling agent.

The present invention provides testosterone-containing pharmaceutical compositions and gels in accordance with the appended claims, and methods for using the same. More particularly, the present invention provides testosterone-containing hydroalcoholic/alcoholic pharmaceutical compositions and gels, suitable for topical and transdermal application, wherein said compositions contain propylene glycol and isopropyl myristate. In addition to achieving a very effective testosterone delivery, the compositions of the invention are aesthetically very attractive. Propylene glycol was shown to be a very potent penetration enhancer in the compositions of the invention, used in combination with isopropyl myristate.

The present invention relates to a pharmaceutical composition comprising testosterone, at least one C2-C6 alcohol, at least one gelling agent, propylene glycol, isopropyl myristate, and water.

Said pharmaceutical composition can be made in various forms, e.g. a gel, a solution, a cream, a lotion, a spray, an ointment, an aerosol. Preferably, said pharmaceutical composition is a gel.

The term "testosterone" as used hereafter refers to testosterone itself and its enantiomers, isomers, tautomers, and chelates. According to the invention, testosterone can be of natural origin, or result from a hemisynthesis or synthesis process.

C2-C6 alcohols are known in the art. Such alcohols encompass ethanol, propanol, isopropanol (propan-2-ol), n-propanol (propan-1-ol), butanol, butan-1-ol, butan-2-ol, ter-butanol, pentanols, hexanols. Ethanol is preferred, since it contributes efficiently towards the transdermal passage of testosterone by evaporating quickly on contact with the skin.

Gelling agents are known in the art. The term 'gelling agent' generally refers to a compound, possibly of polymeric nature, having the capacity to gel when contacted with a specific solvent, e.g. water. Gelling agents make it possible to increase the viscosity of the pharmaceutical compositions according to the invention, but may also act as solubilizing agents. Examples of gelling agents include anionic polymers such as acrylic acid based polymers (including polyacrylic acid polymers, e.g. CARBOPOL® by B.F. Goodrich Specialty Polymers and Chemicals Division of Cleveland, Ohio)., cellulose derivatives, poloxamers and poloxamines, more precisely, Carbomers or acrylic acid-based polymers, e.g. Carbopol® 980 or 940, 981 or 941, 1382 or 1382, 5984, 2984, 934 or 934P (Carbopol® are usually polymers of acrylic acid crosslinked with allyl sucrose or allylpentaerythritol), Pemulen TR1® or TR2 ®, Ultrez, Synthalen CR, etc.); cellulose derivatives such as ethylcelluloses, hydroxypropylcelluloses, hydroxyethylcelluloses, hydroxypropylmethylcelluloses (HPMC), carboxymethylcelluloses (CMC), etc.; poloxamers or polyethylene-polypropylene copolymers such as Lutrol® grade 68 or 127, poloxamines and other gelling agents such as chitosan, dextran, pectins, and natural gums. All of these gelling agents, alone or in combination, may be used in the pharmaceutical composition according to the invention. Said gelling agent can be selected taking into account the pH of the composition according to the invention and the desired viscosity.

Hydroxypropylcellulose, Carbopol® 980 and Lutrol® are particularly preferred in the context of the present invention.

Penetration enhancers are also known in the art. A 'penetration enhancer' is generally an agent known to accelerate the delivery of the drug or active principle through the skin. These agents also have been referred to as penetration accelerants, adjuvants, and absorption promoters. This class of agents includes those with diverse mechanisms of action including those which have the function of improving the solubility and diffusibility of the drug, and those which improve transdermal absorption by changing the ability of the stratum corneum to retain moisture, softening the skin, improving the skin's permeability, acting as penetration assistants or hair-follicle openers or changing temporarily the state of the skin such as the boundary layer.

Unless otherwise stated, percentages (%) refer to amounts by weight based upon total weight of the composition.

According to one aspect, said pharmaceutical composition comprises 0.5 to 5.0 % (w/w), preferably 0.5 to 2.5 % (w/w), more preferably 0.6 to 2.0 % (w/w), even more preferably 0.7 to 1.5 % (w/w), even more preferably 0.75 to 1.25 % (w/w), even more preferably 0.8 to 1.2 % (w/w), and even more preferably 0.9 to 1.1 % (w/w), most preferably about 1.0 % (w/w) of testosterone.

According to another aspect of the invention, said pharmaceutical composition comprises 0.5 to 5.0 % (w/w), preferably 0.5 to 2.5 % (w/w), more preferably 0.75 to 2.25 % (w/w), even more preferably 0.9 to 2.0 % (w/w), even more preferably 1.0 to 1.8 % (w/w), even more preferably 1.25 to 1.75 % (w/w), still more preferably 1.3 to 1.6 % (w/w), most preferably about 1.5 % (w/w) of testosterone.

According to another aspect, said pharmaceutical composition comprises 40.0 to 75.0 % (w/w), preferably, 40.0 to 70.0 % (w/w), more preferably 45.0 to 65.0 % (w/w), even more preferably 50.0 to 60.0 % (w/w), even more preferably 52.0 to 58.0 % (w/w), even more preferably 53.0 to 57.0 % (w/w), still more preferably 54.0 to 56.0 % (w/w), most preferably about 56 % (w/w) of at least one C2-C6 alcohol.

According to another aspect of the invention, said C2-C6 alcohol is selected from the group consisting of ethanol, propan-1-ol and propan-2-ol, and mixtures thereof Preferably said C2-C6 alcohol is ethanol.

According to another aspect of the invention, said pharmaceutical composition comprises 0.1 to 5.0 % (w/w), preferably 0.15 to 4.5 % (w/w), more preferably 0.2 to 4.0 % (w/w), even more preferably 0.25 to 3.5 % (w/w), even more preferably 0.3 to 3.0 % (w/w), even more preferably 0.4 to 2.5 % (w/w), still more preferably 0.5 to 2.0 % (w/w), most preferably about 0.5 to 1.0 % (w/w) of at least one gelling agent. According to another aspect of the invention, said gelling agent is selected from the group consisting of acrylic acid-based polymers, including cellulosics, including cellulose esters and derivatives (cellulose derivatives such as ethylcellulose, hydroxypropylcellulose, hydroxyethylcellulose, hydroxypropylmethylcellulose (HPMC), carboxymethylcellulose (CMC), etc); Carbomers such as Carbopol® polymers, e.g. Carbopol® 980 or 940, 981 or 941, 1382 or 1382, 5984, 2984, 934, or 934P, Pemulen TR1 ® or TR2 ®, Ultrez, Synthalen CR, etc.; poloxamines, poloxamers or polyethylene-polypropylene copolymers such as Lutrol ® grade 68 or 127, poloxamines or other gelling agents such as chitosan, dextran, pectins, and natural gums, and mixtures thereof

According to another aspect of the invention, said pharmaceutical composition comprises 0.1 to 5.0 % (w/w), preferably 0.15 to 4.5 % (w/w), more preferably 0.2 to 4.0 % (w/w), even more preferably 0.3 to 3.5 % (w/w), even more preferably 0.4 to 3.0 % (w/w), still more preferably 0.5 to 2.5 % (w/w), most preferably 0.5 to 2.0 % (w/w) of propylene glycol. Advantageously according to the invention, for the testosterone contents and compositions of the invention, propylene glycol was found to be a very effective and compatible penetration enhancer. In addition, the use of propylene glycol according to the invention leads to pharmaceutical compositions having a very attractive appearance. The pharmaceutical compositions of the invention are very transparent, visually attractive, and have very pleasant texture, which improves the use by a patient. In addition, the pharmaceutical compositions of the invention require lower alcohol contents. Moreover, the compositions of the invention are very stable, and propylene glycol has proven to be compatible with standard neutralizers, e.g. triethanolamine. The compositions according to the invention may include several penetration enhancers.

According to the present invention, said pharmaceutical composition optionally further comprises water.

The present invention provides a pharmaceutical composition comprising:

- 0.5 to 5.0 % (w/w) of testosterone,
- 40.0 to 75.0 % (w/w) of a least one C2-C6 alcohol,
- 0.1 to 5.0 % (w/w) of at least one gelling agent,
- 0.1 to 5.0 % (w/w) of propylene glycol,
- 0.05 to 5.0 % (w/w) of isopropyl myristate,
- optionally, water.

In another embodiment, the present invention provides a pharmaceutical composition comprising:

- 0.75 to 1.25 % (w/w) of testosterone,
- 50.0 to 70.0 % (w/w) of a least one C2-C6 alcohol,
- 0.1 to 4.0 % (w/w) of at least one gelling agent,
- 0.2 to 4.0 % (w/w) of propylene glycol,
- 0.05 to 5.0 % (w/w) of isopropyl myristate,
- optionally, water.

In another embodiment, the present invention provides a pharmaceutical composition comprising:

- 0.8 to 1.2 % (w/w) of testosterone,
- 50.0 to 70.0 % (w/w) of a least one C2-C6 alcohol,
- 0.1 to 3.0 % (w/w) of at least one gelling agent,
- 0.3 to 3.0 % (w/w) of propylene glycol,
- 0.05 to 5.0 % (w/w) of isopropyl myristate,
- optionally, water.

In another embodiment, the present invention provides a pharmaceutical composition comprising:

- 0.9 to 1.1 % (w/w) of testosterone,
- 50.0 to 70.0 % (w/w) of a least one C2-C6 alcohol,
- 0.1 to 2.5 % (w/w) of at least one gelling agent,
- 0.4 to 2.5 % (w/w) of propylene glycol,
- 0.05 to 5.0 % (w/w) of isopropyl myristate,
- optionally, water.

In another embodiment, the present invention provides a pharmaceutical composition comprising:

- about 1.0 % (w/w) of testosterone,
- 50.0 to 70.0 % (w/w) of a least one C2-C6 alcohol,
- 0.1 to 2.5 % (w/w) of at least one gelling agent,
- 0.4 to 2.5 % (w/w) of propylene glycol,
- 0.05 to 5.0 % (w/w) of isopropyl myristate,
- optionally, water.

In another embodiment, the present invention provides a pharmaceutical composition comprising:

- 1.0 to 2.0 % (w/w) of testosterone,
- 50.0 to 75.0 % (w/w) of a least one C2-C6 alcohol,
- 0.1 to 5.0 % (w/w) of at least one gelling agent,
- 0.1 to 5.0 % (w/w) of propylene glycol,
- 0.05 to 5.0 % (w/w) of isopropyl myristate,
- optionally, water.

In another embodiment, the present invention provides a pharmaceutical composition comprising:

- 1.25 to 1.75 % (w/w) of testosterone,
- 50.0 to 70.0 % (w/w) of a least one C2-C6 alcohol,
- 0.1 to 4.0 % (w/w) of at least one gelling agent,
- 0.2 to 4.0 % (w/w) of propylene glycol,
- 0.05 to 5.0 % (w/w) of isopropyl myristate,
- optionally, water.

In another embodiment, the present invention provides a pharmaceutical composition comprising:

- 1.4 to 1.6 % (w/w) of testosterone,
- 50.0 to 70.0 % (w/w) of a least one C2-C6 alcohol,
- 0.1 to 3.0 % (w/w) of at least one gelling agent,
- 0.3 to 3.0 % (w/w) of propylene glycol,
- 0.05 to 5.0 % (w/w) of isopropyl myristate,
- optionally, water.

In another embodiment, the present invention provides a pharmaceutical composition comprising:

- about 1.5 % (w/w) of testosterone,
- 50.0 to 70.0 % (w/w) of a least one C2-C6 alcohol,
- 0.1 to 2.5 % (w/w) of at least one gelling agent,
- 0.4 to 2.5 % (w/w) of propylene glycol,

- 0.05 to 5.0 % (w/w) of isopropyl myristate,
- optionally, water.

Said pharmaceutical composition is suitable for the controlled transdermal delivery of testosterone. Thus, said pharmaceutical composition can be auto-administered by the patient him/herself, and does not require the presence of a physician during administration. Also, the administration does not require surgery or injections. Advantageously according to the invention, propylene glycol acts as a very potent penetration enhancer for testosterone, in particular in combination with the testosterone dosage and the gelling agent(s) of the present composition.

In another aspect, the pharmaceutical composition of the invention further comprises a base. Advantageously, said base is pharmaceutically acceptable, and is preferably selected from the group consisting of triethanolamine, sodium hydroxide, ammonium hydroxide, potassium hydroxide, arginine, aminomethylpropanol or tromethamine, and mixtures thereof Where the pH of said pharmaceutical composition is not optimized for transdermal administration, e.g. where said gelling agent comprises at least one acrylic acid-based polymer, said base contributes to the neutralization of said pharmaceutical composition, for topical application onto human skin. Furthermore, said base (neutralizer) allows optimum swelling of the polymer chains during the neutralization of the charges and the formation of polymer salts. Especially when said gelling agent comprises an acrylic acid-based polymer, said base preferably comprises triethanolamine. It also allows an optimum viscosity to be achieved in the pharmaceutical composition according to the invention. The skilled person would know how to choose a suitable amount of said base in the composition, especially with respect to the nature of said gelling agent present therein, and the alcohol content of the composition, in order to reach the desired final pH in the composition. For example, with carbomers and/or if there is a high alcohol content, one can use tromethamine and/or NaOH as a base, in amounts chosen as to reach the desired final pH in the composition. Alternatively, in one embodiment, the pharmaceutical composition according to the invention comprises 0.1 to 5.0 % (w/w), preferably 0.15 to 4.5 % (w/w), more preferably 0.2 to 4.0 % (w/w), even more preferably 0.25 to 3.5 % (w/w), even more preferably 0.3 to 3.0 % (w/w), even more preferably 0.4 to 2.5 % (w/w), still more preferably 0.5 to 2.0 % (w/w), most preferably 0.5 to 1.0 % (w/w) of triethanolamine.

Preferably, the pH of the pharmaceutical composition according to the invention will be between 2 and 9, preferably between 3 and 8 and even more preferably between 3 and 7. In another aspect of the invention, said pharmaceutical composition further comprises isopropyl myristate. According to the invention, it was unexpectedly shown that addition of isopropyl myristate leads to markedly improved results when used in conjunction with propylene glycol as a further penetration enhancer. Said pharmaceutical composition comprises 0.05 to 5.0 % (w/w), preferably 0.1 to 4.0 % (w/w), more preferably 0.15 to 3.0 % (w/w), even more preferably 0.25 to 2.5 % (w/w), even more preferably 0.25 to 2.0 % (w/w), even more preferably 0.25 to 1.5 % (w/w), still more preferably 0.25 to 1.0 % (w/w), most preferably 0.5 to 0.75 % (w/w) of isopropyl myristate.

Accordingly, in one embodiment, the present invention provides a pharmaceutical composition comprising:

- 0.5 to 5.0 % (w/w) of testosterone,
- 40.0 to 75.0 % (w/w) of a least one C2-C6 alcohol,
- 0.1 to 5.0 % (w/w) of at least one gelling agent,
- 0.1 to 5.0 % (w/w) of propylene glycol,
- 0.1 to 4.0 % (w/w) of isopropyl myristate,
- optionally, water.

In another embodiment, the present invention provides a pharmaceutical composition comprising:

- 0.75 to 1.25 % (w/w) of testosterone,
- 50.0 to 70.0 % (w/w) of a least one C2-C6 alcohol,
- 0.1 to 4.0 % (w/w) of at least one gelling agent,
- 0.2 to 4.0 % (w/w) of propylene glycol,
- 0.15 to 2.0 % (w/w) of isopropyl myristate,
- optionally, water.

In another embodiment, the present invention provides a pharmaceutical composition comprising:

- 0.8 to 1.2 % (w/w) of testosterone,
- 50.0 to 70.0 % (w/w) of a least one C2-C6 alcohol,
- 0.1 to 3.0 % (w/w) of at least one gelling agent,
- 0.3 to 3.0 % (w/w) of propylene glycol,
- 0.35 to 1.5 % (w/w) of isopropyl myristate,

- optionally, water.

In another embodiment, the present invention provides a pharmaceutical composition comprising:

- 0.9 to 1.1 % (w/w) of testosterone,
- 50.0 to 70.0 % (w/w) of a least one C2-C6 alcohol,
- 0.1 to 2.5 % (w/w) of at least one gelling agent,
- 0.4 to 2.5 % (w/w) of propylene glycol,
- 0.4 to 1.0 % (w/w) of isopropyl myristate,
- optionally, water.

In another embodiment, the present invention provides a pharmaceutical composition comprising:

- about 1.0 % (w/w) of testosterone,
- 50.0 to 70.0 % (w/w) of a least one C2-C6 alcohol,
- 0.1 to 2.5 % (w/w) of at least one gelling agent,
- 0.4 to 2.5 % (w/w) of propylene glycol,
- about 0.5 % (w/w) of isopropyl myristate,
- optionally, water.

In another embodiment, the present invention provides a pharmaceutical composition comprising:

- 1.0 to 2.0 % (w/w) of testosterone,
- 50.0 to 75.0 % (w/w) of a least one C2-C6 alcohol,
- 0.1 to 5.0 % (w/w) of at least one gelling agent,
- 0.1 to 5.0 % (w/w) of propylene glycol,
- 0.1 to 4.0 % (w/w) of isopropyl myristate,
- optionally, water.

In another embodiment, the present invention provides a pharmaceutical composition comprising:

- 1.25 to 1.75 % (w/w) of testosterone,
- 50.0 to 70.0 % (w/w) of a least one C2-C6 alcohol,
- 0.1 to 4.0 % (w/w) of at least one gelling agent,
- 0.2 to 4.0 % (w/w) of propylene glycol,
- 0.15 to 2.0 % (w/w) of isopropyl myristate,
- optionally, water.

In another embodiment, the present invention provides a pharmaceutical composition comprising:

- 1.4 to 1.6 % (w/w) of testosterone,
- 50.0 to 70.0 % (w/w) of a least one C2-C6 alcohol,
- 0.1 to 3.0 % (w/w) of at least one gelling agent,
- 0.3 to 3.0 % (w/w) of propylene glycol,
- 0.35 to 1.5 % (w/w) of isopropyl myristate,
- optionally, water.

In another embodiment, the present invention provides a pharmaceutical composition comprising:

- about 1.5 % (w/w) of testosterone,
- 50.0 to 70.0 % (w/w) of a least one C2-C6 alcohol,
- 0.1 to 2.5 % (w/w) of at least one gelling agent,
- 0.4 to 2.5 % (w/w) of propylene glycol,
- 0.4 to 1.0 % (w/w) of isopropyl myristate,
- optionally, water.

Additionally, said pharmaceutical composition may comprise usual pharmaceutical additives, including salt(s), emollient(s), stabilizer(s), antimicrobial(s), fragrance(s), and/or propellant(s). Said pharmaceutical composition may also include at least one further active ingredient, e.g. another hormone.

The invention also provides a gel useful for transdermal or transcutaneous delivery comprising said pharmaceutical composition according to the invention. Thus, the invention is also directed to a testosterone-containing hydroalcoholic gel.

According to another embodiment, the present invention provides a dose packet, unit dose packet or multiple dose packet containing said pharmaceutical composition or said hydroalcoholic gel. Advantageously, such conditioning of said pharmaceutical composition renders application easier for a patient. Thus, these forms of packaging can reflect the schedule of application, e.g. daily or weekly administration. According to another embodiment, there is provide a dispenser, e.g. with hand pump or valve, containing said pharmaceutical composition or said hydroalcoholic gel. Such dispensers allow for flexibility in the administered dosage, as a function of the amount of composition to be applied.

According to one embodiment, said packets or dispensers can be accompanied by a notice giving instructions for the use thereof.

The pharmaceutical compositions, gels, packets and containers of the invention are useful for treating testosterone deficiency, and treating and/or preventing testosterone deficiency-related conditions and/or disorders.

The term 'treat' or 'treatment' as used herein refers to any treatment of a mammalian condition, disorder, or disease associated with a depressive disorder, and includes, but is not limited to, preventing the condition, disorder, or disease from occurring in a subject which may be predisposed to the condition, disorder, or disease, but has not yet been diagnosed as having the condition, disorder, or disease; inhibiting the condition, disorder, or disease, for example, arresting the development of the condition, disorder, or disease; relieving the condition, disorder, or disease, for example, causing regression of the condition, disorder, or disease; or relieving the condition caused by the disease or disorder, for example, stopping the symptoms of the disease or disorder.

The term 'prevent' or 'prevention', in relation to a condition, disorder, or disease, means no condition, disorder, or disease development if none had occurred, or no further condition, disorder, or disease development if there had already been development of the condition, disorder, or disease.

As used herein, 'testosterone deficiency' refers to lower serum levels of free testosterone in a subject as compared to the median serum levels for healthy subject of the same age. For example, normal cycling women produce approximately 300 $\mu$g of testosterone per day. Their total serum testosterone levels generally range from about 20 ng/dL to about 80 ng/dL averaging about 40 ng/dL. In healthy young women, for example, mean free testosterone levels are generally about 3.6 pg/mL. However, several factors may influence both total and free testosterone serum levels. For example, in regularly ovulating women, there is a small but significant increase in plasma testosterone levels during the middle third of the menstrual cycle. However, mean testosterone levels (1.2 nmol/L or 33 ng/dL) and mean free testosterone levels (12.8 pmo/L or 3.6 pg/mL) during the luteal and follicular phases are not significantly different. Additionally, testosterone production declines continuously after age 30 so that serum testosterone levels in a 60-year-old woman are only 50% of the levels in a young 30-year-old woman. Although the percentage of free testosterone generally does not vary with age, an absolute decline in free testosterone has been observed. This decline does not occur abruptly at menopause but instead occurs gradually and continuously as a result of the age-related decrease in both the adrenal and ovarian androgen production. Thus, women begin to experience symptoms associated with menopause in the immediate pre-menopausal years. The decline in testosterone following menopause results from the combination of ovarian failure, decreasing renal secretion, and peripheral conversion. Also, for example, after ovariectomy, testosterone concentrations decrease by about 50%. Diagnosis of a testosterone deficiency is known to the average physician practicing in the relevant field of medicine.

The pharmaceutical compositions and gels of the present invention are also useful to treat a number of physiological and psychological parameters and/or conditions associated with testosterone deficiency in a man or a woman. For example, the pharmaceutical compositions and gels of the present invention are useful for:

- increasing libido and improving sexual performance and/or treating sexual dysfunction; normalizing hypogonadism;
- increasing bone mineral density and related markers, increasing lean body mass and decreasing fat body mass, improving muscle mass and performance;
- normalizing glucose levels; improving diabetic retinopathy as well as lowering the insulin requirements of diabetic subjects; treating, preventing or reducing the onset of cataracts;
- treating, preventing or reducing the onset of cardiovascular disease, including normalizing hypertension, and treating obesity; normalizing cholesterol levels; normalizing abnormal electrocardiograms of subjects and treating, preventing or reducing vasomotor symptoms;
- preventing human immunodeficiency virus wasting syndrome;
- preventing osteoporosis, osteopenia, vaginal dryness, and thinning of the vaginal wall; relieving menopausal symptoms and hot flashes; treating premenstrual syndrome;
- treating, preventing or reducing the onset of Alzheimer's disease, dementia;
- improving cognition, improving mood and self-esteem, treating and/or preventive depressive disorders; treating, preventing or reducing cognitive dysfunction;

- treating autoimmune diseases.

The present pharmaceutical compositions and gels can also be used in "combination therapy" with another hormone or steroid, or a pharmaceutical agent that increases testosterone levels in a subject, or an estrogenic hormone, or another pharmaceutical agent such as, for example, an antidepressant agent.

The invention also relates to a process for preparing a pharmaceutical composition, e.g. in the form of a gel or a solution, according to the invention.

In one aspect, said process comprises the step of dissolving testosterone, with stirring, in a solvent of at least one C2-C6 alcohol and propylene glycol, and isopropyl myristate.

In another aspect, said process comprises the step of adding water, with stirring, to the mixture obtained.

Where the preparation of a gel is desired, at least one gelling agent, such as Carbopol®, is then added to the mixture, with stirring.

Optionally, a base/neutralizer such as triethanolamine is added to the mixture, with stirring.

In another aspect, the invention relates to a process for preparing a pharmaceutical composition or a gel according to the invention, comprising the steps of:

- preparing a mixture containing at least one C2-C6 alcohol, propylene glycol, isopropyl myristate and testosterone;
- optionally adding water, and mixing,
- adding a gelling agent to this mixture, and mixing;
- optionally adding a base, and mixing again.

In another aspect, the invention also relates to the use of the gel or the solution according to the invention for the preparation of a medicinal product for transdermal application for the treatment of a physiological condition associated with an androgen/testosterone deficiency.

In another aspect, the present invention relates to the gel or the solution according to the invention for use in a method of treatment.

In one embodiment, said method comprises the step of administering testosterone, to a subject in need thereof

According to one aspect, the pharmaceutical composition or the gel of the present invention is applied on healthy skin, e.g. to the shoulder, or to the external part of the arm, thigh or leg.

In order to measure and determine the amount of testosterone to be delivered to a subject in need thereof, serum testosterone concentrations can be measured using standard assay techniques.

Therefrom, the skilled person would know how to determine the amount of composition to be administered, depending upon the exact testosterone dosage of said pharmaceutical composition.

In general, the usual daily dose of the pharmaceutical composition in the form of a gel or a solution according to the invention is between 2.5 g and 5 g of the formulation per day.

The advantages of the invention will become apparent from the following examples, which are given below as mere illustrations, and are non limitative.

The skilled person will appreciate that the present invention can incorporate any number of the preferred features described above.

The practice of the present invention will employ, unless otherwise indicated, conventional techniques of pharmacology and pharmaceutics, which are within the skill of the art.

## Examples

### Example 1

Studies on transcutaneous delivery of testosterone gel formulations were carried out on animal and human skin using radio-labelled ($^{14}$C) testosterone and liquid scintillation titration. These studies give access to kinetics parameters (released amount after 24h and flux). Two aspects were investigated:

- Physical-chemistry studies (testosterone solubility and gel viscosity)
- Transcutaneous delivery (influence of testosterone concentration, nature of gelling agent, presence of penetration enhancers)

**Material and methods**

**1. Material**

Chemicals

Testosterone (Fluka)
$^{14}$C testosterone (Amersham)

Gelling agents

Carbopol® 934P (Carb. 934P) (Polyplastic)
Carbopol® 980 (Carb. 980) (Lot 4419, Laboratoires Besins Iscovesco)
Klucel®
Neutralizer
Triethanolamine (lot 4343, Laboratoires Besins Iscovesco)

Penetration enhancers

Transcutanol (Gattefossé)
Oleic acid (OA) (Prolabo)
Propylene glycol (PG) (Gattefossé)
Propylene glycol dipelargonate (PGDP) (Gattefossé)
Animal skin model
Male naked rat, 5 weeks

Human skin model

Biopsies from abdominal plastics, white female, 35 years old

**2. Preparation of radio-labeled testosterone gels**

The gelling agent (polymer) is swelled overnight in the required amount of water. Labeled and non-labeled testosterone is dissolved in ethanol, possibly with penetration enhancer. The aqueous and alcoholic phases are mixed, then neutralized with triethanolamine. Mixing is carried out with a spatula until perfect homogeneity is reached. Seventeen formulations were evaluated for transcutaneous delivery. The composition thereof is given in the table below (in % wt based on total weight of fomulation). Formulations 13 and 14 correspond to non-gelled solutions.

Table 1

| Nr | Testosterone | Gelling agent | 95% EtOH | Tri-ethanolamine | PG | OA | isopropyl myristate |
|----|--------------|---------------|----------|------------------|----|----|---------------------|
| 11 | 1 | 1% Carb. 934 P | 58 | 1.35 | 0 | 0 | 0 |
| 12 | 0.5 | 1% Carb. 934 P | 58 | 1.35 | 0 | 0 | 0 |
| 13 | 0.5 | 0 | 58 | 0 | 0 | 0 | 0 |
| 14 | 0.75 | 0 | 58 | 0 | 0 | 0 | 0 |
| 15 | 2.5 | 1% Carb. 934 P | 58 | 1.35 | 0 | 0 | 0 |
| 16 | 2.5 | 1% Carb. 934 P | 58 | 1.35 | 5 | 0 | 0 |

(continued)

| Nr | Testosterone | Gelling agent | 95% EtOH | Tri-ethanolamine | PG | OA | isopropyl myristate |
|----|--------------|---------------|----------|------------------|----|----|---------------------|
| 17 | 2.5 | 2% Klucel | 58 | 1.35 | 0 | 0 | 0 |
| 18 | 2.5 | 2% Klucel | 58 | 1.35 | 5 | 0 | 0 |
| 19 | 2.5 | 1% Carb. 934 P | 58 | 1.35 | 0 | 0 | 5 |
| 20 | 2.5 | 0.5% Carb. 980 | 58 | 1.35 | 0 | 0 | 0 |
| 21 | 1 | 0.5% Carb. 980 | 58 | 0.6 | 0 | 0 | 0 |
| 22 | 1 | 0.5% Carb. 980 | 58 | 0.6 | 2 | 0 | 0 |
| 23 | 1 | 1% Carb. 980 | 58 | 1.35 | 2 | 0 | 0 |
| 24 | 1 | 1% Carb. 934 P | 58 | 1.35 | 2 | 0 | 0 |
| 25 | 1 | 1% Carb. 934 P | 58 | 1.35 | 2 | 2 | 0 |
| 26 | 1 | 0.5% Carb. 980 | 58 | 0.5 | 1 | 0 | 0 |
| 27 | 1 | 0.5% Carb. 980 | 58 | 0.5 | 1 | 0 | 0.5 |
| (Carb. = Carbopol® ) | | | | | | | |

### 3. Dermal permeation kinetics protocol

#### 3.1 Dermal permeation cell

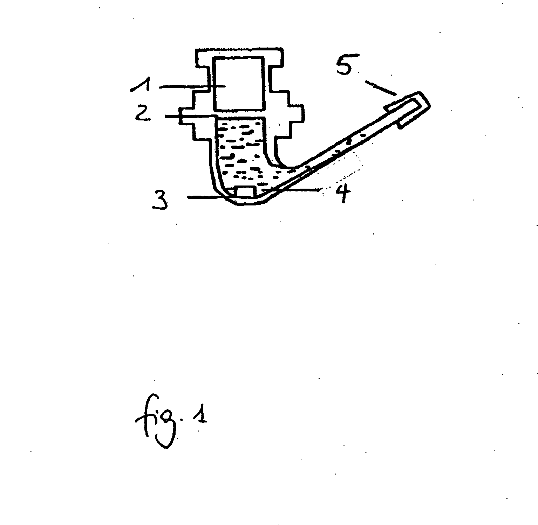

Static flux cells comprising two unequal compartments are used (Figure 1).

An upper cylindrical (section: 2.54 cm$^2$) donor compartment 1 allows the application of a formulation (either solution or topical form) onto the skin 2. This upper compartment 1 is assembled to the lower compartment 4 with a metal ring.

A lower receiving compartment 4 (mean volume 10mL) has an upper opening of 2.54 cm$^2$ as well. A lateral shunt 5, equipped with a needle and catheter system, allows sampling. Homogenization of the medium is this lower compartment is achieved with a magnetic stirrer 3. (See Figure 1.)

#### 3.2 Biological membrane

Animal skin

Skin biopsies of male naked rat skin (IOPS rats, 5 weeks) are sampled at the ventral level. Fat tissues located to the inner face are removed, and the skin is cut in order to obtain samples with an area greater than 2.54 cm$^2$. Each sample is stuck to the lower compartment with the skin external face upwards.

Human skin

Experiments are carried out on surgical plastics sampled at the abdomen level from a white 35 year old female. Samples are used immediately after sampling and dermatomed on a thickness of about 250$\mu$m.

#### 3.3 Study protocol

The two compartments 1, 4 of the dermal permeability cell are assembled with the skin fragment 2. The receiving compartment 4 is filled with a precisely measured volume of a 0.5% albumin solution. The cell is then placed for 15h in a thermostatic bath at 37°C on a stirring platform in order to obtain equilibrium between the skin and the receiving medium.

Kinetics starts after application of the formulation onto the skin 2. Applications are about 20mg on 2.54cm$^2$ of skin. Over a period of 24h, 2mL samples are carried out from the receiving compartment 4 at evenly spaced time points, and 2mL fresh albumin solution is immediately added to the compartment in order to keep its volume constant. For each formulation, five permeation cells are used. After cold-storing, the samples are titrated by liquid scintillation.

### 4. $^{14}$C testosterone titration

The samples are split into two 1mL fractions to which 4mL scintillation liquid (Pico Fluor 40®) are added, metering time is 5min.

Samples are then titrated by liquid scintillation using a β spectrophotometre (Beckman). For scintillation, quenching leads to a decreased detection efficiency, which is accurately evaluated establishing a calibration curve termed quenching curve. For each sample, the metering yield depending upon quenching is calculated using an external standard source and the actual radioactivity is determined.

### Results

### 1. Testosterone solubility

A known amount of solvent (2mL) is stirred at 37°C in a closed Erlenmeyer. Starting from a known amount of teststerone, fractions are progressively added. The remaining amount of testosterone is weighted in order to determine the amount in the Erlenmeyer (by difference).

Testosterone solubility at 37°C in water/ethanol mixtures and various penetration enhancers such as propylene glycol (PG), oleic acid (OA), propylene glycol dipelargonate (PGDP), transcutol, are determined by the increment method.

Table 2

| Testosterone solubility in various vehicles | | | | | |
|---|---|---|---|---|---|
| Solvent | PG (100%) | PG/OA (50/50) | OVA (100%) | PGDP (100%) | Transcutol (100%) |
| Solubility (mg/mL) | 86 | 79 | 66 | 18 | 140 |

Testosterone solubility is also evaluated as a function of ethanol content.

Table 3

| Testosterone solubility in water / ethanol mixtures | | |
|---|---|---|
| 95% Ethanol (v/v) | Testosterone solubility | |
| | mg/mL | g/g |
| 42 / 58 | 14 | 0.015 |
| 53 / 47 | 20 | 0.023 |
| 56 / 44 | 24 | 0.027 |
| 63 / 37 | 30 | 0.035 |
| 74 / 26 | 52 | 0.061 |
| 84 / 16 | 120 | 0.146 |
| 86 / 14 | 160 | 0.195 |
| 100 / 0 | 340 | 0.420 |
| See plots on Figure 2 | | |

### 2. Gel viscosity

Gel rheology was characterized with a Carri-Med controlled-stress rheometer, measurement geometry: plane/plane, diameter 2cm, gap 1mm, temperature 20°C. Rheograms are plotted in flow following a defined shear cycle as follows:

-   up phase: stress increasing from 0 to 300N/m$^2$ in 2min;
-   plateau: constant stress 300N/m$^2$ for 1 min;
-   down phase : stress decreasing from 300N/m$^2$ to 0 in 2min.

All gels had a shear softening (pseudo-plastic) behavior, without any hysteresis cycle.
Two rheological studies were carried out:

- influence of ethanol content on Carbopol®934 gel viscosity
- influence of polymer content on Carbopol®980 gel viscosity.

2.1 Carbopol®934P hydroalcoholic gel viscosity

The influence of ethanol content on Carbopol® hydroalcoholic gels is studied by varying from 50% to 70% the content in 95% ethanol.

Table 4

| Ethanol (% wt) | Viscosity (Pa.s) | Maximal velocity gradient ($s^{-1}$) |
|---|---|---|
| 50 | 15.00 | 22.5 |
| 55 | 6.500 | 50 |
| 60 | 2.375 | 140 |
| 65 | 0.800 | 400 |

Above 55% ethanol, viscosity is smaller, with a strong variation as a function of ethanol content. Viscosity is increased for 50% ethanol.

2.2 Carbopol® 980 gel viscosity

The influence of polymer content on hydroalcoholic gels with 40% ethanol is studied by varying Carbopol®980 content from 0.3% to 0.7%.
Viscosities are measured in the plateau under $300N/m^2$ stress for 1min.

Table 5

| Carbopol® 980 (%) | Viscosity (Pa.s) | Maximal velocity gradient (s-1) |
|---|---|---|
| 0.3 | 0.125 | 1400 |
| 0.4 | 0.610 | 500 |
| 0.5 | 3.50 | 90 |
| 0.6 | 6.10 | 50 |
| 0.7 | 12 | 30 |

**3. Dermal permeation of testosterone formulations on rat skin**

Dermal permeation for the formulations from Table 1 is evaluated in vitro on rat skin. Formulation 25 cannot be evaluated due to heterogeneity (phase separation). Results for released amount at 24h, and released rate at 24h are given in the table below.

Table 6

| Formulation Number | Testosterone (%) | Released amount (24h) ($\mu g/cm^2$) | Released rate (24h) (%) |
|---|---|---|---|
| 11 | 1 | 28.1 | 35 |
| 12 | 0.5 | 11.6 | 29 |
| 13 | 0.5 | 24.4 | 61 |
| 14 | 0.75 | 36.7 | 61 |
| 15 | 2.5 | 16.2 | 8 |
| 16 | 2.5 | 30.9 | 15 |

(continued)

| Formulation Number | Testosterone (%) | Released amount (24h) ($\mu$g/cm$^2$) | Released rate (24h) (%) |
|---|---|---|---|
| 17 | 2.5 | 10.5 | 5 |
| 18 | 2.5 | 36.9 | 18 |
| 19 | 2.5 | 44.5 | 22 |
| 20 | 2.5 | 18.5 | 9 |
| 21 | 1 | 21.6 | 27 |
| 22 | 1 | 33.5 | 42 |
| 23 | 1 | 34.4 | 43 |
| 24 | 1 | 36.9 | 46 |
| 25 | 1 | - | - |
| 26 | 1 | 30.0 | 37.5 |
| 27 | 1 | 62.0 | 77 |
| Formulations 11-27: 8mg/cm$^2$ are deposited. | | | |

### 4. Testosterone permeation on human skin

Formulations 26 and 27 are evaluated on human skin, together with formulation 15 as a reference. Results are detailed below:

Formulation 26

Mean values for permeation parameters on 5 cells:

| Time (h) | Amount Q ($\mu$g) | Amount/area Q/S ($\mu$g/cm$^2$) | Q/S standard deviation (n=5) |
|---|---|---|---|
| 2 | 0 | 0 | 0 |
| 4 | 1.9 | 0.75 | 0.4 |
| 6 | 3.5 | 1.4 | 0.6 |
| 9 | 5.6 | 2.2 | 0.6 |
| 12 | 7.4 | 2.9 | 0.8 |
| 24 | 15.3 | 6.01 | 1.6 |
| Average kinetics plot is given on Fig. 3. Mean flux: 0.26 $\mu$g/cm$^2$.h | | | |

Formulation 27

Mean values for permeation parameters on 5 cells:

| Time (h) | Amount Q ($\mu$g) | Amount/area Q/S ($\mu$g/cm$^2$) | Q/S standard deviation (n=5) |
|---|---|---|---|
| 2 | 0 | 0 | 0 |
| 4 | 3 | 1.2 | 0.9 |
| 6 | 5.6 | 22.2 | 1.1 |
| 9 | 8.6 | 3.4 | 1.2 |
| 12 | 11.7 | 4.6 | 1.4 |

(continued)

| Time (h) | Amount Q ($\mu$g) | Amount/area Q/S ($\mu$g/cm$^2$) | Q/S standard deviation (n=5) |
|---|---|---|---|
| 24 | 22.1 | 8.7 | 2.5 |
| Average kinetics plot is given on Fig. 4.<br>Mean flux: 0.39 $\mu$g/cm$^2$.h | | | |

Formulation 15 (reference)

Mean values for permeation parameters on 5 cells:

| Time (h) | Amount Q ($\mu$g) | Amount/area Q/S ($\mu$g/cm$^2$) | Q/S standard deviation (n=5) |
|---|---|---|---|
| 2 | 0 | 0 | 0 |
| 4 | 1.1 | 0.45 | 0.3 |
| 6 | 2.2. | 0.85 | 0.5 |
| 9 | 6.6 | 1.3 | 0.6 |
| 12 | 4.3 | 1.7 | 0.7 |
| 24 | 9.1 | 3.6 | 1.2 |
| Average kinetics plot is given on Fig. 5.<br>Mean flux: 0.15 $\mu$g/cm$^2$.h | | | |

**Example 2** (for illustrative purposes)

**Material and methods**

**1. Material**

[1, 2, 6, 7-$^3$H] **testosterone** (Amersham Laboratories): MW=295, solution at 1 mCi/mL (37 MBq/mL) in ethanol; specific activity 99Ci/mmol (3.66 TBq/mmol), i.e. 336 mCi/mg (lot 18).

Absolute ethanol    Carlo Erba    lot V4N051154N

Material controlled by Laboratoires Besins

Carbopol® 980          lot 92010/4420
Carbopol®934            lot /3898
Testosterone            lot 92039/4461
Isopropyl myristate     lot 91169/5781
Triethanolamine (TEA)   lot 9304020/5721
Propylene glycol (PG)

Formulations to be applied onto skin: (in weight for 100g final formulation).

| Fmltn | Testosterone | Gelling agent | Absolute ethanol | neutralizer | propylene glycol | isopropyl myristate |
|---|---|---|---|---|---|---|
| 1 (ref.) | 2.5 | Carb.934: 1.0 | 57 | TEA: 1.35 | - | - |

(continued)

| Fmltn | Testosterone | Gelling agent | Absolute ethanol | neutralizer | propylene glycol | isopropyl myristate |
|-------|--------------|---------------|------------------|-------------|------------------|---------------------|
| 2 | 1 | Carb.980: 0.6 | 58 | TEA: 0.60 | 0.5 | - |
| 3 | 1 | Carb.980: 0.8 | 58 | TEA: 0.80 | 0.5 | - |
| 4 | 1 | Carb.980: 0.9 | 67 | NaOH (0.1M): 4.725 | - | 0.5 |
| 5 | 1 | Carb.980: 0.5 | 67 | TEA: 0.50 | - | 0.5 |

Formulations are prepared as follows:

Formulation 1:

1g of radio-labelled gel is prepared as follows.

- 100μL (100μCi) of the radio-labelled testosterone solution ($^3$H) are dried and resuspended with:
- 595mg of a solution containing 2.5g testosterone in 57g absolute ethanol,
- 391.5mg base gel, obtained by swelling 1g Carbopol®934 in 38.15g water; after complete swelling and before use, the mucilage is homogenized a few seconds with ultra-turax.
- 13.5mg TEA.

Radioactive concentration of formulation: 100μCi/g of gel
i.e. 0.8μCi and 200μg testosterone for a loading of 8mg (about 10μL) per cell of 1.77 cm$^2$

Formulation 2:

1g of radio-labelled gel is prepared according to the following protocol:

- 100μL (100μCi) of the radio-labelled testosterone solution ($^3$H) are dried and resuspended with:
- 595mg of a solution containing 1g testosterone and 0.5g PG in 58g absolute ethanol,
- 393mg base gel, obtained by swelling 0.6g Carbopol®980 in 38.7g water; after complete swelling and before use, the mucilage is homogenized a few seconds with ultra-turax.
- 12mg of TEA half diluted in water.

Radioactive concentration of formulation: 100μCi/g of gel
i.e. 0.8μCi and 80μg testosterone for a loading of 8mg (about 10μL) per cell

Formulation 3:

1g of radio-labelled gel is prepared according to the following protocol:

- 100μL (100μCi) of the radio-labelled testosterone solution ($^3$H) are dried and resuspended with:
- 595mg of a solution containing 1g testosterone and 0.5g PG in 58g absolute ethanol,
- 389mg base gel, obtained by swelling 0.8g Carbopol®980 in 38.1g water; after complete swelling and before use, the mucilage is homogenized a few seconds with ultra-turax.
- 16mg of TEA half diluted in water.

Radioactive concentration of formulation: 100μCi/g of gel
i.e. 0.8μCi and 80μg testosterone for a loading of 8mg (about 10μL) per cell

Formulation 4:

1g of radio-labelled gel is prepared according to the following protocol:

- 100μL (100μCi) of the radio-labelled testosterone solution ($^3$H) are dried and resuspended with:
- 685mg of a solution containing 1g testosterone and 0.5g isopropyl myristate in 67g absolute ethanol,
- 267.75mg base gel, obtained by swelling 0.9g Carbopol®980 in 25.875g water; after complete swelling and before use, the mucilage is homogenized a few seconds with ultra-turax.
- 47.25mg of NaOH (0.1M).

Radioactive concentration of formulation: 100μCi/g of gel
i.e. 0.8μCi and 80μg testosterone for a loading of 8mg (about 10μL) per cell

Formulation 5:

1g of radio-labelled gel is prepared according to the following protocol:

- 100μL (100μCi) of the radio-labelled testosterone solution ($^3$H) are dried and resuspended with:
- 685mg of a solution containing 1g testosterone and 0.5g isopropyl myristate in 67g absolute ethanol,
- 305mg base gel, obtained by swelling 0.5g Carbopol®980 in 30g water; after complete swelling and before use, the mucilage is homogenized a few seconds with ultra-turax.
- 10mg of TEA half diluted in water.

Radioactive concentration of formulation: 100μCi/g of gel
i.e. 0.8μCi and 80μg testosterone for a loading of 8mg (about 10μL) per cell

### 2. Methods

**In vitro dermal absorption:**

Principle

In vitro transdermal absorption is quantitatively studied on human ventral dermatomed biopsies placed in a static diffusion cell (Franz cell), which allows contacting dermis with a survival liquid (receptor fluid) in which absorption through skin is to be dosed.

Cell

A dermal biopsy is maintained horizontally between two parts of the cell, thus delimiting two compartments:

- one epidermal compartment is comprised of a glass cylinder, having a precisely defined area of 1.77cm$^2$, placed on the upper side of the skin;
- the other, dermal, applied to the lower face of the tegument, comprises a reservoir of fixed volume carrying a lateral collection port.

The two elements are assembled via a clamp.
The lower compartment (dermal) is filled with a survival liquid constituted of a sodium chloride solution at 9g/L supplemented with bovine serum albumin at 15g/L. At each time point, the survival liquid is entirely sampled out by the lateral collection port and is replaced by fresh liquid.
The lower part of the cell is thermostated at 37°C. Homogeneity of the temperature and the content in the receptor fluid, is maintained by stirring (magnetic stirrer).

The upper part (epidermal compartment) is open towards the exterior, thus exposing the epidermal surface to the air in the laboratory.

Preparation of human ventral dermatomed skin dermal biopsies:

These are samples from human ventral skin from plastic surgery from white donors. Skin is kept at -20°C before use. Adherent sub-dermal fat is removed with a scalpel, and skin is brought to a thickness of about 0.5mm with a dermatome. For each assay, the various skin samples are equally distributed between the lots.

**General protocol**

Franz cells are usually installed the day before loading the formulation to be studied. The epidermal compartment is contacted with the atmosphere in the laboratory, the dermal compartment is thermostated to 37°C and the skin is contacted with albuminated physiological serum for about 17 hours. Under these conditions, the skin is highly hydrated.

10$\mu$L solution (about 8$\mu$L, about 8$\mu$Ci) are applied with a micropipette onto the whole of the surface of the epidermis delimited by the glass cylinder. Sampling from the liquid contained in the dermal compartment are carried out via the lateral collection port at time points 2h, 4h, 6h, 8h, and 24h. For each time point, the survival liquid is entirely sampled out and replace by fresh liquid.

At the end of the assay, the treated dermal surface is washed with 200$\mu$L of various solvents according to the following protocol:

- 1st wash: Cetavlon ®/water (1/9, v/v);
- 2nd wash: water;
- 3rd wash: Cetavlon ®/water (1/9, v/v);
- 4th wash: wash;
- 5th wash: water.

The application area is then wiped with a Q-tip®.

The epidermis and the dermis are then mechanically separated with a scalpel, and digested in respectively in 1mL and 3mL Soluene® (Packard).

**Radioactivity measurements**

Detection is carried out by liquid scintillation using a particle counter Packard-tricarb 4530.

Preparation of radioactive samples:

The survival liquid sampled from the lower compartment of the diffusion cells is directly incorporated in 15mL of liquid scintillation cocktail (Picofluor 40R, Packard) and metered for radioactivity measurement.

Liquids from washes and Q-tips® are introduced in a vial containing about 30mL 95°Ethanol and weighted exactly. After incubating overnight at 4°C, the radioactivity of an exactly weighted aliquot from this dilution is determined following the same procedure as for the survival liquids.

Epidermis and dermis must first be dissolved in strong organic bases. They are dissolved by contacting 24h at 37°C with Soluene 350® Packard (1mL per 100 mg tissue) to which 15mL liquid scintillation cocktail (Hionic Fluor 30®, Packard) are added, and then metered.

Radioactivity measurements:

Metering rate is corrected, as far as quenching is concerned, by the method of the external calibration, in order to obtain disintegrations per minute (dpm) accounting for the real activity of each sample. The background is deducted for each sample in cpm. For each scintillation liquid, a specific quenching curve is established.

Results are expressed in weight or percentage of substance found in the samples with respect to the administered amount, determined from the metering rates of suitably diluted calibrations.

Transdermal absorption:

Intensity of transdermal absorption is evaluated by computing the absorption percentage (%) of the loaded amount (Qi) as a function of time:

$$\% = (Qt / Qi) \times 100$$

wherein Qt is the absorbed amount at time point t.

Average flux and absorbed amount for each time interval are also determined and expressed respectively in ng/cm$^2$/h and in ng.

Average results correspond to 6-8 experimental determinations and are associated to the standard deviation (Sd).

Comparison of the average results is carried out by variance analysis.

**3. Results**

Concentrations expressed as percentages are by weight of substance for 100g of final formulation.

In vitro testosterone penetration in Franz cells (Cumulated values)

Average values +/- Sd as a function of time

| Fmltn | Values | Survival liquid in the cell | | | | |
|---|---|---|---|---|---|---|
| | | 2h | 4h | 6h | 8h | 24h |
| **1** | **% time** | **0.23%** | **0.42%** | **0.54%** | **0.63%** | **0.97%** |
| | +/- Sd | 0.08% | 0.12% | 0.13% | 0.15% | 0.24% |
| | | | | | | |
| n=6 | **amount (ng)** | **501** | **902** | **1184** | **1378** | **2113** |
| | +/- Sd | 169 | 254 | 278 | 322 | 518 |
| | | | | | | |
| **2** | **% time** | **0.40%** | **0.77%** | **1.03%** | **1.23%@** | **1.98%@** |
| | +/- Sd | 0.18% | 0.29% | 0.36% | 0.41% | 0.59% |
| | | | | | | |
| n=8 | **amount (ng)** | **351** | **666** | **892** | **1067** | **1721** |
| | +/- Sd | 159 | 256 | 317 | 360 | 508 |
| **3** | **% time** | **0.35%** | **0.72%** | **0.97%** | **1.17%** | **1.94%@** |
| | +/- Sd | 0.21% | 0.34% | 0.41% | 0.47% | 0.59% |
| | | | | | | |
| n=8 | **amount (ng)** | **313** | **639** | **868** | **1047** | **1735** |
| | +/- Sd | 188 | 302 | 370 | 418 | 530 |
| | | | | | | |
| **4** | **% time** | **0.52%** | **0.94%@** | **1.21%@** | **1.40%@** | **2.17%*** |
| | +/- Sd | 0.15% | 0.27% | 0.34% | 0.38% | 0.58% |
| | | | | | | |
| n=7 | **amount (ng)** | **462** | **834** | **1071** | **1243** | **1927** |
| | +/- Sd | 133 | 237 | 301 | 341 | 514 |
| | | | | | | |
| **5** | **% time** | **0.56%** | **1.04%@** | **1.33%@** | **1.53%@** | **2.47%*** |
| | +/- Sd | 0.49% | 0.71% | 0.78% | 0.81% | 0.99% |

(continued)

| Fmltn | Values | Survival liquid in the cell | | | | |
|---|---|---|---|---|---|---|
| | | 2h | 4h | 6h | 8h | 24h |
| n=7 | amount (ng) | 484 | 899 | 1153 | 1324 | 2138 |
| | +/- Sd | 424 | 615 | 673 | 707 | 861 |

@: different from reference (variance analysis, Fisher test significant with p<5%)
* : different from reference (anova; Fisher and Scheffe F test, significant with p<5%)

<u>Retrieved amounts</u> (Cumulated values)

| Fmltn | Values | Amounts at 24h | | | | | |
|---|---|---|---|---|---|---|---|
| | | E | D | E+D | C+D | W | T |
| 1 | % | 36.35% | 0.98% | 37.33% | 1.95% | 47.03% | 85.33% |
| | +/- Sd | 8.15% | 0.55% | | | 9.69% | |
| n=6 | amount (ng) | 79072 | 2131 | 81203 | 4244 | 102306 | 185622 |
| | +/- Sd | 18516 | 1193 | | | 21075 | |
| 2 | % | 44.36%@ | 1.55% | 45.91% | 3.53% | 36.06% | 83.95% |
| | +/- Sd | 8.44% | 1.42% | | | 5.23% | |
| n=8 | amount (ng) | 38513 | 1349 | 39862 | 3069 | 31301 | 72844 |
| | +/- Sd | 7325 | 1235 | | | 4539 | |
| 3 | % | 40.56% | 1.91% | 42.47% | 3.85% | 41.47% | 85.88% |
| | +/- Sd | 4.98% | 1.02% | | | 6.93% | |
| n=8 | amount (ng) | 36200 | 1701 | 37900 | 3436 | 37015 | 76650 |
| | +/- Sd | 4443 | 916 | | | 6189 | |
| 4 | % | 44.56%@ | 2.10% | 46.66% | 4.27% | 31.77% | 80.60% |
| | +/- Sd | 5.37% | 0.65% | | | 4.99% | |
| n=7 | amount (ng) | 39506 | 1865 | 41371 | 3792 | 28164 | 71462 |
| | +/- Sd | 4763 | 577 | | | 4419 | |
| 5 | % | 29.27%° | 2.97%* | 32.24% | 5.44% | 46.58% | 81.29% |
| | +/- Sd | 7.35% | 1.18% | | | 8.18% | |

(continued)

| Fmltn | Values | Amounts at 24h | | | | | |
|---|---|---|---|---|---|---|---|
| | | E | D | E+D | C+D | W | T |
| n=7 | amount (ng) | 25393 | 2580 | 27973 | 4718 | 40397 | 70508 |
| | +/- Sd | 6374 | 1022 | | | 7096 | |

E: epidermis, D: dermis, C: cell, W: washes, T: total

@: different from reference (variance analysis, Fisher test significant with p<5%)

°: different from formulation 2, 3 and 4 (anova, Fisher test, p<5%)

*: different from reference (anova; Fisher and Scheffe F test, significant with p<5%)

In vitro testosterone penetration in Franz cells (Non cumulated values)

Average values +/- Sd as a function of time:

| Fmltn | Values | Survival liquid in the cell | | | | |
|---|---|---|---|---|---|---|
| | | 0-2h | 2-4h | 4-6h | 6-8h | 8-24h |
| 1 n=6 | % | 0.23% | 0.18% | 0.13% | 0.09% | 0.34% |
| | +/- Sd | 0.08% | 0.05% | 0.04% | 0.03% | 0.12% |
| | | | | | | |
| | amount (ng) | 501 | 401 | 281 | 194 | 735 |
| | +/- Sd | 169 | 101 | 91 | 59 | 256 |
| | | | | | | |
| | Flux (ng/cm$^2$/h) | 141.60 | 113.34 | 79.41 | 54.88 | 25.95 |
| | +/- Sd | 47.71 | 28.65 | 25.78 | 16.53 | 9.03 |
| | | | | | | |
| 2 n=8 | % | 0.40% | 0.36%@ | 0.26%@ | 0.20%* | 0.75%* |
| | +/- Sd | 0.18% | 0.13% | 0.09% | 0.07% | 0.22% |
| | | | | | | |
| | amount (ng) | 351 | 315 | 226 | 174 | 654 |
| | +/- Sd | 159 | 113 | 82 | 63 | 194 |
| | | | | | | |
| | Flux (ng/cm$^2$/h) | 99.14 | 88.97 | 63.98 | 49.24 | 23.10 |
| | +/- Sd | 45.03 | 31.91 | 23.13 | 17.74 | 6.86 |
| | | | | | | |
| 3 n=8 | % | 0.35% | 0.36%@ | 0.26%@ | 0.20%* | 0.77%* |
| | +/- Sd | 0.21% | 0.15% | 0.09% | 0.07% | 0.21% |
| | | | | | | |
| | amount (ng) | 313 | 325 | 229 | 179 | 688 |
| | +/- Sd | 188 | 136 | 84 | 61 | 184 |
| | | | | | | |
| | Flux (ng/cm$^2$/h) | 88.45 | 91.94 | 64.82 | 50.68 | 24.29 |

(continued)

| Fmltn | Values | Survival liquid in the cell | | | | |
|---|---|---|---|---|---|---|
| | | 0-2h | 2-4h | 4-6h | 6-8h | 8-24h |
| | +/- Sd | 53.09 | 38.33 | 23.86 | 17.25 | 6.49 |
| | | | | | | |
| | % | 052% | 0.42%@ | 0.27%@ | 0.19%@ | 0.77%* |
| | +/- Sd | 0.15% | 0.15% | 0.09% | 0.06% | 0.21% |
| | | | | | | |
| 4 | amount (ng) | 462 | 372 | 237 | 172 | 684 |
| | +/- Sd | 133 | 132 | 81 | 50 | 185 |
| n=7 | | | | | | |
| | Flux (ng/cm$^2$/h) | 130.51 | 105.15 | 67.00 | 48.49 | 24.16 |
| | +/- Sd | 37.61 | 37.22 | 22.82 | 14.26 | 6.53 |
| | | | | | | |
| | % | 0.56%@ | 0.48%* | 0.29%* | 0.20%@ | 0.94%* |
| | +/- Sd | 0.49% | 0.23% | 0.08% | 0.05% | 0.20% |
| | | | | | | |
| 5 | amount (ng) | 484 | 415 | 254 | 171 | 814 |
| | +/- Sd | 424 | 198 | 73 | 46 | 177 |
| | | | | | | |
| n=7 | Flux (ng/cm$^2$/h) | 136.86 | 117.14 | 71.74 | 48.22 | 28.53 |
| | +/- Sd | 116.80 | 55.98 | 20.67 | 13.04 | 6.26 |
| @: different from reference (variance analysis, Fisher test significant with $p < 5\%$) *: different from reference (variance analysis, Fisher test and Scheffe F-test significant with $p < 5\%$) | | | | | | |

**Claims**

1. Pharmaceutical composition comprising:

   - 0.5 to 5.0 % (w/w) of testosterone,
   - 40.0 to 75.0 % (w/w) of a least one C2-C6 alcohol,
   - 0.1 to 5.0 % (w/w) of at least one gelling agent,
   - 0.1 to 5.0 % (w/w) of propylene glycol,
   - 0.05 to 5.0 % (w/w) of isopropyl myristate,
   - optionally, water.

2. Pharmaceutical composition according to claim 1, wherein said C2-C6 alcohol is selected from the group consisting of ethanol, propan-1-ol and propan-2-ol, and mixtures thereof.

3. Pharmaceutical composition according to any one of claims 1-2, wherein said gelling agent is selected from the group consisting of acrylic acid-based polymers, carboxymethylcelluloses, hydroxypropylcelluloses, hydroxyethylcelluloses, cellulosics, and mixtures thereof.

4. Pharmaceutical composition according to any one of claims 1-3, wherein said gelling agent comprises at least one gelling agent selected from the group consisting of carbomers, e.g. Carbopol® 980, Carbopol® 934P, Carbopol® 1342, Carbopol® 1382, and mixtures thereof.

**5.** Pharmaceutical composition according to any one of claims 1-4, further comprising a base, preferably selected from the group consisting of triethanolamine, sodium hydroxide, ammonium hydroxide, potassium hydroxide, arginine, aminomethylpropanol, tromethamine, and mixtures thereof.

**6.** Gel useful for transdermal or transcutaneous delivery comprising a pharmaceutical composition according to any one of claims 1-5.

**7.** Dose packet, unit dose packet or multiple dose packet containing a pharmaceutical composition according to any one of claims 1-5 or a gel according to claim 6.

**8.** Dispenser, e.g. with hand pump, containing a pharmaceutical composition according to any one of claims 1-5 or a gel according to claim 6.

**9.** Process for preparing a pharmaceutical composition according to any one of claims 1-5 or a gel according to claim 6, comprising the steps of:

- preparing a mixture containing at least one C2-C6 alcohol, propylene glycol, isopropyl myristate and testosterone;
- optionally adding water, and mixing;
- adding a gelling agent to this mixture, and mixing;
- optionally adding a base, and mixing again.


**Patentansprüche**

**1.** Pharmazeutische Zusammensetzung, umfassend

- 0,5 bis 5,0 % (Gew./Gew.) Testosteron,
- 40,0 bis 75,0 % (Gew./Gew.) mindestens eines C2-C6-Alkohols,
- 0,1 bis 5,0 % (Gew./Gew.) mindestens eines Geliermittels,
- 0,1 bis 5,0 % (Gew./Gew.) Propylenglycol,
- 0,05 bis 5,0 % (Gew./Gew.) Isopropylmyristat,
- wahlweise Wasser.

**2.** Pharmazeutische Zusammensetzung gemäß Anspruch 1, wobei der C2-C6-Alkohol ausgewählt ist aus der Gruppe Ethanol, Propan-1-ol und Propan-2-ol und deren Gemische.

**3.** Pharmazeutische Zusammensetzung gemäß irgendeinem der Ansprüche 1 bis 2, wobei das Geliermittel ausgewählt ist aus der Gruppe Polymere auf Acrylsäure-Basis, Carboxymethylcellulosen, Hydroxypropylcellulosen, Hydroxyethylcellulosen, Cellulosehaltige und deren Gemische.

**4.** Pharmazeutische Zusammensetzung gemäß irgendeinem der Ansprüche 1 bis 3, wobei das Geliermittel mindestens ein Geliermittel umfasst, ausgewählt aus der Gruppe Carbomere, z.B. Carbopol® 980, Carbopol® 934P, Carbopol® 1342, Carbopol® 1382 und deren Gemische.

**5.** Pharmazeutische Zusammensetzung gemäß irgendeinem der Ansprüche 1 bis 4, zudem umfassend eine Base, vorzugsweise ausgewählt aus der Gruppe Triethanolamin, Natriumhydroxyd, Ammoniumhydroxyd, Kaliumhydroxyd, Arginin, Aminomethylpropanol, Tromethamin und deren Gemische.

**6.** Gel, nützlich für transdermale oder transkutane Verabreichung, umfassend eine pharmazeutische Zusammensetzung aus irgendeinem der Ansprüche 1 bis 5.

**7.** Dosispacket, Einheitsdosispacket oder Mehrfachdosispacket, enthaltend eine pharmazeutische Zusammensetzung aus irgendeinem der Ansprüche 1 bis 5 oder ein Gel aus Anspruch 6.

**8.** Spender, z.B. mit Handpumpe, enthaltend eine pharmazeutische Zusammensetzung aus irgendeinem der Ansprüche 1 bis 5 oder ein Gel aus Anspruch 6.

**9.** Herstellungsverfahren für eine pharmazeutische Zusammensetzung aus irgendeinem der Ansprüche 1 bis 5 oder ein Gel aus Anspruch 6, umfassend die Schritte

- Herstellen eines Gemischs, enthaltend mindestens einen C2-C6-Alkohol, Propylenglycol, Isopropylmyristat und Testosteron;
- wahlweise Zusetzen von Wasser, und Vermischen;
- Zusetzen eines Geliermittels zum Gemisch, und Vermischen;
- wahlweise Zusetzen einer Base, und nochmaliges Vermischen.

**Revendications**

**1.** Une composition pharmaceutique contenant :

- 0,5 à 5,0% (poids/poids) de testostérone,
- 40,0 à 75,0% (poids/poids) d'au moins un alcool en C2-C6,
- 0,1 à 5,0% (poids/poids) d'au moins un agent gélifiant,
- 0,1 à 5,0% (poids/poids) de propylène glycol,
- 0,05 à 5,0% (poids/poids) de myristate d'isopropyle,
- éventuellement, de l'eau.

**2.** La composition pharmaceutique selon la revendication 1, où ledit alcool en C2-C6 est sélectionné dans le groupe se composant d'éthanol, propan-1-ol et propan-2-ol, et des mélanges de ceux-ci.

**3.** La composition pharmaceutique selon l'une quelconque des revendications 1 à 2, où ledit agent gélifiant est sélectionné dans le groupe se composant de polymères à base d'acide acrylique, carboxyméthylcelluloses, hydroxypropylcelluloses, hydroxyéthylcelluloses, produits cellulosiques, et des mélanges de ceux-ci.

**4.** La composition pharmaceutique selon l'une quelconque des revendications 1 à 3, où ledit agent gélifiant contient au moins un agent gélifiant sélectionné dans le groupe se composant de carbomères, par exemple Carbopol® 980, Carbopol® 934P, Carbopol® 1342, Carbopol® 1382, et des mélanges de ceux-ci.

**5.** La composition pharmaceutique selon l'une quelconque des revendications 1 à 4, contenant en outre une base sélectionnée de préférence dans le groupe se composant de triéthanolamine, hydroxyde de sodium, hydroxyde d'ammonium, hydroxyde de potassium, arginine, aminométhyl propanol, trométhamine, et des mélanges de ceux-ci.

**6.** Un gel utile pour une administration transdermique ou transcutanée contenant une composition pharmaceutique selon l'une quelconque des revendications 1 à 5.

**7.** Un paquet en doses, un paquet en doses unitaires ou un paquet en doses multiples contenant une composition pharmaceutique selon l'une quelconque des revendications 1 à 5 ou un gel selon la revendication 6.

**8.** Un distributeur, par exemple avec une pompe à main, contenant une composition pharmaceutique selon l'une quelconque des revendications 1 à 5 ou un gel selon la revendication 6.

**9.** Un processus de préparation d'une composition pharmaceutique selon l'une quelconque des revendications 1 à 5 ou un gel selon la revendication 6, comprenant les opérations suivantes :

- la préparation d'un mélange contenant au moins un alcool en C2-C6, propylène glycol, myristate d'isopropyle et testostérone,
- éventuellement l'ajout d'eau, et une opération de mélange,
- l'ajout d'un agent gélifiant à ce mélange, et une opération de mélange,
- éventuellement l'ajout d'une base, et à nouveau une opération de mélange.

fig. 1

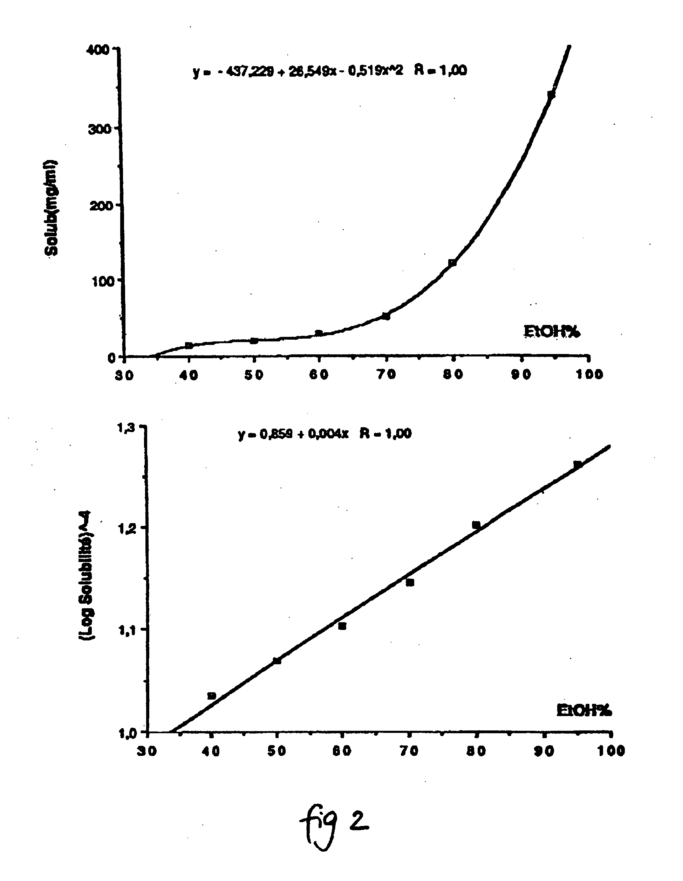

fig 2

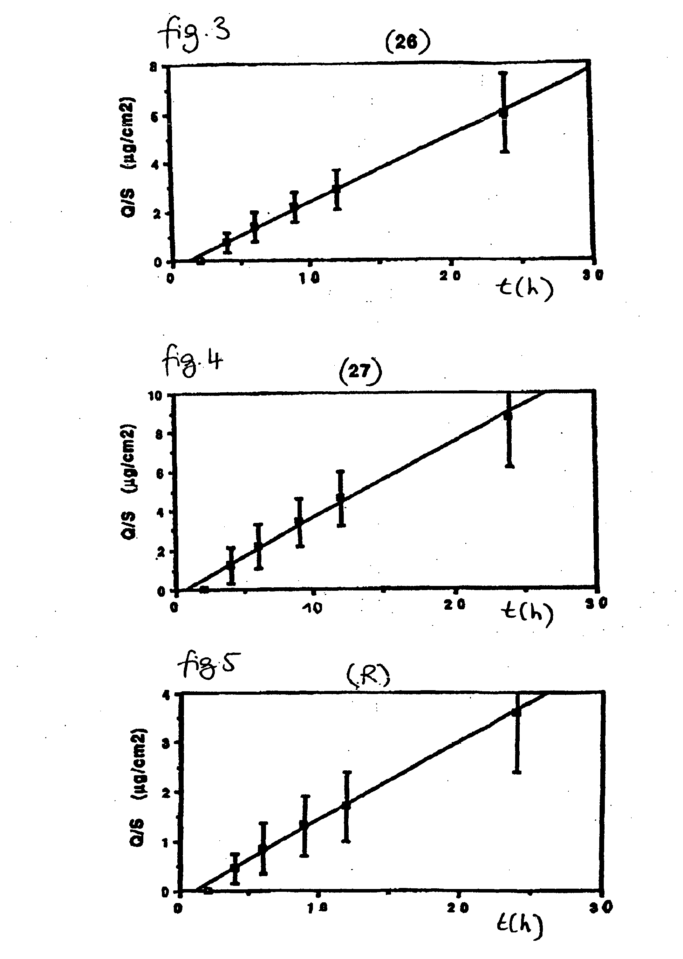

fig.3

(26)

fig.4

(27)

fig.5

(R)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2003088974 A
- WO 2002051421 A
- WO 1999020257 A
- WO 2003047548 A